# EUROPEAN PATENT APPLICATION

(11) **EP 1 029 544 A1**
(43) Date of publication of application: **23.08.2000**
(21) Application number: 98947915.9
(22) Date of filing: 15.10.1998
(51) Int. Cl.: A61K 31/70, A61K 47/48

(54) **INTRA-CANCER-CELL NUCLEASE ACTIVATOR**

(30) Priority: 16.10.1997 JP 28396897
(71) Applicant: Nippon Shinyaku Co., Ltd., Kyoto-shi Kyoto 601-8550 (JP)
(72) Inventor: HIRABAYASHI, Kazuko, Room 307, Greenpalace, Ukyo-ku, Kyoto-shi, Kyoto 616-8105 (JP); SEKI, Junzo, Ibaraki-shi, Osaka 567-0032 (JP)
(74) Representative: Strych, Werner Maximilian Josef, Dr.
(86) International application number: JP9804695
(87) International publication number: WO9920283

(57) **Abstract**

A drug efficacious for cancer therapy and a novel drug containing a double-stranded RNA such as poly(I).poly(C). Specifically, an intra-cancer-cell nuclease activator containing 2-O-(2-diethylaminoethyl)-carbamoyl-1,3-O-dioleylglycerol and a composite comprising a carrier prepared from a phospholipid as an essential component and poly(I).poly(C) or mismatched poly(I).poly(C).

## Description

### TECHNICAL FIELD

The present invention relates to a cancer cell nuclease activator.

The term "cancer cell nuclease activator" as used in this specification means a drug which activates the nucleases in cancer cells and thereby induce apoptosis and, hence, death of the cancer cells. Also as used herein, the code "I" stands for inosinic acid, "C" for cytidylic acid, "A" for adenylic acid, and "U" for uridylic acid.

The terms "mismatched poly(I)· poly(C) and mismatched poly(A)· poly(U) mean the poly(I)· poly(C) and poly(A)· poly(U) which contain non-complementary nucleic acid bases among those constituting a double strand as is well known in the art.

### BACKGROUND ART

Poly(I)·poly(C) is a double-stranded RNA comprising a polyribonucleotide copolymer of polyinosinic acid and polycytidylic acid, and is known to be a medicinally active substance having potent interferon-inducing activity and immunopotentiating activity. The fact that poly(I)· poly(C) has immunopotentiating activity suggests that the substance will indirectly inhibit growth of cancer cells through immune reactions, thus prompting many researchers to explore into its potential utility as a therapeutic drug for malignant tumors. However, the immunity-mediated indirect action of poly(I)· poly(C) is not potent enough to inhibit growth of cancer cells and an anticancer therapy with poly(I)·poly(C) has not been implemented as yet. Therapeutic regimens using poly(I)· poly(C) for other indications based on its interferon-inducing and immunopotentiating activities have not been developed, either.

Poly(A)·poly(U), which is a polyribonucleotide copolymer of polyadenylic acid and polyuridylic acid, mismatched poly(I)·poly(C), and mismatched poly(A)·poly(U) are also considered to have similar activities, through varying in degrees.

Meanwhile, as an effective carrier for intracellular delivery of drugs, there is known carriers generally called cationic liposomes such as Lipofectin (registered trademark) as well as a carrier comprising a glycerol derivative such as 2-O-(2-diethylaminoethyl)carbaxmoyl-1,3-O-dioleoylglycerol of the following chemical formula [I] and a phospholipid as essential components (e.g. PCT WO91/17424, PCT WO94/19314].

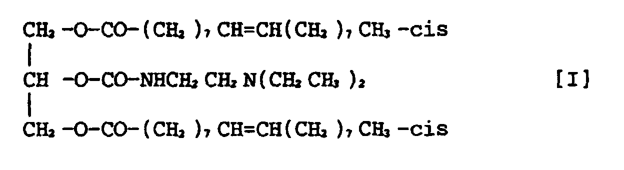

The cationic liposome is visualized as a small vesicle having a lipid bilayer structure and assuming a positive charge in aqueous solution. Since such a cationic liposome is positively charged and a double-stranded RNA such as poly(I)·poly(C) is negatively charged in aqueous solution, the cationic liposome and poly(I)·poly(C), for instance, may easily form a complex.

However, it was not known at all whether the very double-stranded RNA, e.g. poly(I)·poly(C), or the complex thereof with a cationic liposome would be able to activate the nucleases in cancer cells to thereby induce apoptosis and, hence, death of the cancer cells.

### DISCLOSURE OF INVENTION

The object of the present invention is to provide an effective drug for cancer therapy. The invention has for its object also to provide a novel drug containing a double-stranded RNA such as poly(I)·poly(C).

The inventors of the present invention found after much research that a cancer cell nuclease activating agent is effective in the therapy of malignant tumors and accordingly have developed the present invention.

The present invention, therefore, is directed to a cancer cell nuclease activator. Whether a substance is a cancer cell nuclease activator can be easily determined experimentally, for example by observing fragmenttion of a DNA or RNA as in Test Example 2 which is presented hereinafter. Typically, the invention encompasses a cancer cell nuclease activating composition comprising a complex of a carrier effective for intracellular delivery of a drug substance with a member selected from the group consisting of poly(I)·poly(C), mismatched poly(I)·poly(C), poly(A)·poly(U), and mismatched poly(A)·poly(U) (those double-stranded RNAs will hereinafter be referred to each and collectively as "poly(I)·poly(C) or equivalent" and a cancer cell nuclease activating composition comprising a complex of a cationic liposome with poly(I)· poly(C) or equivalent.

The preferred embodiment of the invention includes a cancer cell nuclease activating composition comprising a complex (briefly, the complex) of a carrier (briefly, the carrier) comprising 2-O-(2-diethylaminoethyl)carbamoyl-1,3-O-dioleoylglycerol (hereinafter referred to as the glycerol derivative) and a phospholipid as essential components with poly(I)·poly (C) or equivalent (the composition will hereinafter be referred to as the activator of the invention).

The preferred exemplary activator of the invention is now described in detail.

The carrier may be generally regarded as a cationic liposome but need not necessarily be strictly in the form of a cationic liposome only provided that it is functionally qualified to deliver a drug substance into cells.

The chain length of poly(I)·poly(C) or equivalent according to the present invention is not particularly restricted but, taking poly(I)·poly(C) as an example, it is suitable to employ one in the range of 50-2,000 base pairs (bp). An RNA from 100 to 500 bp is preferred and one from 200-400 bp is still more preferred. If the chain length is less than 50 bp, the RNA will not be sufficiently effective, while use of one with a chain length of over 2,000 bp may present a safety problem. The poly(I)·poly(C) within the range of 100-5 00 bp is considered to be an RNA balanced in efficacy and safety. Since the poly(I)·poly(C) or equivalent usually exists with a given distribution of various chain lengths, the above-mentioned chain length of poly(I)·poly(C) is a mean chain length.

The phospholipid for use in the present invention is not particularly restricted provided that it is a pharmaceutically acceptable phospholipid, thus including but not limited to phosphatidylcholine, phosphatidylethanolamine, phosphatidylinositol, phosphatidylserine, sphingomyelins, and lecithin. Hydrogenated phospholipids can also be used. The preferred phospholipid includes egg yolk phosphatidylcholine, egg yolk lecithin, soybean lecithin, and egg yolk phosphatide. Two or more different phospholipids can also be used in combination. Compared with phosphatidylethanolamine which is commonly used in cationic liposomes, phosphatidylcholine and lecithin are conducive to a significant attenuation of toxicity without compromise in activity.

The ratio of the carrier to poly(I)·poly(C) or equivalent in the complex depends on the kinds of phospholipid and poly(I)·poly(C) or equivalent, the type of cancer, and other factors but the recommended proportion of poly(I)·poly(C) relative to 10 weight parts of the carrier is 0.05-10 weight parts, preferably 0.1-4 weight parts, and more preferably 0.5-2 weight parts.

The ratio of the glycerol derivative to the phospholipid in the carrier depends on the kind and amount of poly(I)·poly(C) or equivalenta nd the kind of phospholipid but the recommended proportion of phospholipid relative to each weight part of the glycerol derivative is 0.1-10 weight parts, preferably 0.5-5 weight parts, and more preferably 1-2 weight parts.

The activator of the invention may for example be provided in the form of a liquid preparation (an injection or a drip infusion), in which the complex is dispersed in aqueous solution, or a lyophilizate thereof. In the case of a liquid preparation, the recommended concentration of the complex is 0.001-25% (w/v), preferably 0.01-5% (w/v), and more preferably 0.1-1% (w/v).

The activator of the invention may contain pharmaceutically acceptable additives, such as an auxiliary emulsifier, stabilizer, isotonizing agent, and/or a pH control agent, in suitable amounts. Specifically, auxiliary emulsifiers such as C₆₋₂₂ fatty acids (e.g. caprylic acid, capric acid, lauric acid, myristic acid, palmitic acid, stearic acid, oleic acid, linoleic acid, arachidonic acid, docosahexenoic acid), their pharmaceutically acceptable salts (e.g. sodium salts, potassium salts, calcium salts, etc.), albumin, dextran, etc., stabilizers such as cholesterol, phosphatidine, etc., isotonizing agents such as sodium chloride, glucose, maltose, lactose, sucrose, trehalose, etc., and pH control agents such as hydrochloric acid, sulfuric acid, phosphoric acid, acetic acid, sodium hydroxide, potassium hydroxide, triethanolamine, etc. can be mentioned.

The activator of the present invention can be produced by the general technology for production of liposomes. A typical method comprises mixing a predetermined amount of water (e.g. water for injection, distilled water for injection, or physiological saline) with a predetermined amount of the glycerol derivative and phospholipid under stirring, dispersing the mixture with a suitable dispersing machine such as a homomixer, homogenizer, ultrasonic disperser, ultrasonic homogenizer, high-pressure emulsifier-disperser, Microfluidizer (tradename), Nanomizer (tradename), Ultimizer (tradename), or Manton-Gaulin high-pressure homogenizer, then adding a predetermined amount of poly(I)·poly(C) or equivalent, and redispersingt he mixture to provide the activator of the invention for injection. The optional additives mentioned above can be added at a suitable stage before or after dispersing. As an alternative, the activator of the invention can be produced by adding water to a ternary mixture of the glycerol derivative, phospholipid, and poly(I)·poly(C) or equivalent and dispersing the whole mixture. Moreover, a crude dispersion step may be interposed.

The activator thus prepared by the above dispersing procedure may be freeze-dried to provide a lyophilized activator of the invention. This freeze-drying operation can be carried out in the conventional manner. For example, the activator of the invention as obtained by said dispersing procedure is sterilized and distributed into vials. The filled vials are subjected to preliminary freezing at about -40-20°C for about 2 hours and, then, to primary drying in vacuo at about 0 -10°C and further to secondary drying in vacuo at about 15-25°C. Generally, the vials are purged with nitrogen gas and stoppered to provide the objective lyophilized activator of the invention.

The lyophilized stabilizer of the invention can be generally reconstituted by adding a suitable solvent (for reconstitution) and put to use. The solvent for reconstitution includes water for injection, physiological saline, and other ordinary infusions. The volume of the solvent for reconstitution varies with the intended use and is not particularly restricted but may preferably be 0.5-2 times the volume prior to freeze-drying, or not larger than 500 mL.

The activator of the invention activates cancer cell nucleases to induce apoptosis and death of cells and is only sparingly toxic so that it finds application with dramatic efficacy in the therapy of cancer, for example hepatocarcinoma, in mammals inclusive of man. Particularly, the activator containing a complex formed between the carrier and poly(I)·poly(C) is highly efficacious and yet is very low in toxicity.

The activator of the invention can be administered intravenously, topically, transmucosally, and by other routes in the treatment of neoplastic diseases. When the activator of the invention is used for the therapy of hepatocarcinoma, it is preferably administered intravenously, into the hepatic artery, or into the portal vein.

The dosage of the activator of the invention in cancer therapy depends on the kinds of poly(I)·poly(C) or equivalent and phospholipid,t he type of cancer, the stage of cancer, the recipient's age and species, route of administration, and treatment modality, among other conditions. In terms of poly(I)·poly(C) or equivalent, the recommended dosage is usually 50 µg-50 mg/man per dose and preferably 100 µg-2 mg/man per dose. In terms of poly(I)·poly(C) as such, the recommended dosage is usually 50 µg-50 mg/man per dose and preferably 100 µg-2 mg/man per dose. The activator of the invention can be administered in one shot or by drip injection once through 3 times a day, every day, every other day, or on a weekly or fortnightly basis.

### EXAMPLES

The following working examples and test examples illustrate the present invention in further detail. It should be understood that the concentration of the activator of the invention is invariably expressed in the concentration of said poly(I)·poly(C) in the activator.

### Example 1

A solution of 40 g of maltose in 100 mL of water for injection was mixed with 2 g of the glycerol derivative and 2 g of purified egg yolk lecithin and the mixture was treated with a homogenizer for 5 minutes to prepare a crude carrier dispersion. This crude dispersion was further treated with a bench-top compact emulsifier-disperser for 1 hour and adjusted to 250 mL with water for injection. The resulting carrier dispersion was recovered. To 250 mL of this carrier dispersion was added 150 mL of an aqueous solution containing 500 mg of poly(I)·poly(C) [mean chain length : about 200 bp] with stirring, and using a bench-top compact emulsifier-disperser, the mixture was further treated for 1 hour to provide the activator of the invention. This activator was then distributed into vials, 1 mL per vial, and freeze-dried in the conventional manner.

### Example 2

A solution of 4 kg of sucrose in 10 L of water for injection was mixed with 50 g of the glycerol derivative and 30 mg of egg yolk phosphatide and the mixture was treated with a Manton-Gaulin high-pressure homogenizer for 10 minutes. The resulting dispersion was made up to 25 L with water for injection and recovered. To 20 L of this carrier dispersion was added 12 L of an aqueous solution containing 10 g of poly(I)·poly(C) [mean chain length : about 200 bp] with stirring, a nd the mixture was adjusted to pH 5.5 with hydrochloric acid and further treated with a Manton-Gaulin high-pressure homogenizer for 30 minutes to provide the activator of the invention. This activator was then distributed into vials, 20 mL per vial, and freeze-dried in the conventional manner to provide a lyophilizate. This lyophilized stabilizer was reconstituted by adding a commercial 5% glucose infusion (500 mL).

### Example 3

A solution of 20 g of glucose in 100 mL of water for injection was mixed with 2 g of the glycerol derivative and 2 g of soybean lecithin and the mixture was treated with a homogenizer for 5 minutes to prepare a crude carrier dispersion. This crude dispersion was further treated with a bench-top compact emulsifier-disperser for 1 hour and adjusted to 250 mL with water for injection. The resulting carrier dispersion was recovered. To 250 mL of this carrier dispersion was added 150 mL of an aqueous solution containing 50 mg of poly(I)·poly(C) [mean chain l ength : about 200 bp] with stirring, and using a bench-top compact emulsifier-disperser, the mixture was further treated for 1 hour to provide the activator of the invention.

### Example 4

A solution of 40 g of maltose in 100 mL of water for injection was mixed with 1.2 g of the glycerol derivative and 2.0 g of purified egg yolk lecithin and this crude dispersion was further treated with a bench-top compact emulsifier-disperser for 30 minutes and made up to 250 mL with water for injection. The resulting carrier dispersion was recovered. To 250 mL of this carrier dispersion was added 150 mL of an aqueous solution containing 200 mg of poly(I)·poly(C) [mean chain length : about 200 bp] with stirring, and using a bench-top compact emulsifier-disperser, the mixture was further treated for 2 hours to provide the activator of the invention.

### Example 5

A solution of 40 g of maltose in 100 mL of water for injection was mixed with 1.2 g of the glycerol derivative and 2.0 g of purified egg yolk lecithin and this crude dispersion was further treated with a bench-top compact emulsifier-disperser for 30 minutes and made up to 250 mL with water for injection. The resulting carrier dispersion was recovered. To 250 mL of this carrier dispersion was added 150 mL of an aqueous solution containing 100 mg of poly(I) [mean chain length : 360 base] and 100 mg of poly (C) [mean chain length : 318 base] with stirring, and using a bench-top compact emulsifier-disperser, the mixture was further treated for 2 hours to provide the activator of the invention.

### Example 6

A solution of 40 g of maltose in 100 mL of water for injection was mixed with 2 g of the glycerol derivative and 2 g of purified egg yolk lecithin and the mixture was treated with a homogenizer for 5 minutes to prepare a crude carrier dispersion. This crude dispersion was further treated with a bench-top compact emulsifier-disperser for 1 hour and adjusted to 250 mL with water for injection. The resulting carrier dispersion was recovered. To 250 mL of this carrier dispersion was added 150 mL of an aqueous solution containing 250 mg of poly(I) [mean chain length : 1419 base] and 250 mg of poly(C) [mean chain length : 1491 base] with stirring, and using a bench-top compact emulsifier-disperser, the mixture was further treated for 1 hour to provide the activator of the invention. This activator was then distributed into vials, 1 mL per vial, and freeze-dried in the conventional manner.

### Example 7

A solution of 40 g of maltose in 100 mL of water for injection was mixed with 1.2 g of the glycerol derivative and 2.0 g of purified egg yolk lecithin and this crude dispersion was further treated with a bench-top compact emulsifier-disperser for 30 minutes and made up to 250 mL with water for injection. The resulting carrier dispersion was recovered. To 250 mL of this carrier dispersion was added 150 mL of an aqueous solution containing 100 mg of poly(I) [mean chain length : 84 base] and 100 mg of poly (C) [mean chain length : 76 base] with stirring, and using a bench-top compact emulsifier-disperser, the mixture was further treated for 2 hours to provide the activator of the invention.

### Example 8

The activator of the invention containing poly(I)·poly(C) [mean chain length : about 350 bp] was prepared in the same manner as given in Example 4.

### Example 9

The activator of the invention containing poly(I)·poly(C) [mean chain length : about 1450 bp] was prepared in the same manner as given in Example 4.

### Example 10

The activator of the invention containing poly(I)·poly(C) [mean chain length : about 80 bp] was prepared in the same manner as given in Example 4.

### Test Example 1: Growth inhibitory effect on various cell lines (in vitro)

A 96-well plate was seeded with cells at a density of 10⁴ cells/well. On the following day, the activator of Example 4 or adriamycin was added and the cultivation was continued. After 3 days the viable cell count was determined by the MTT method. The results are shown in Tables 1 and 2.

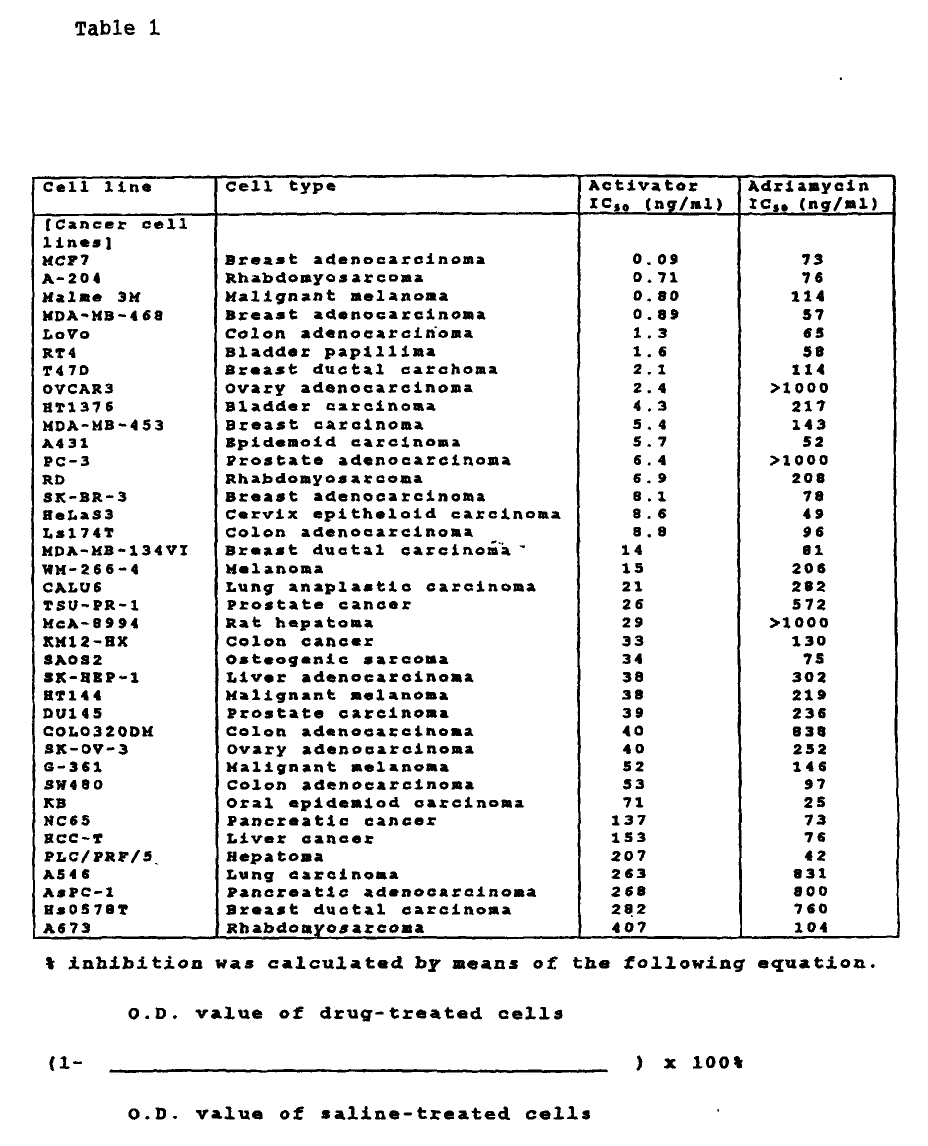

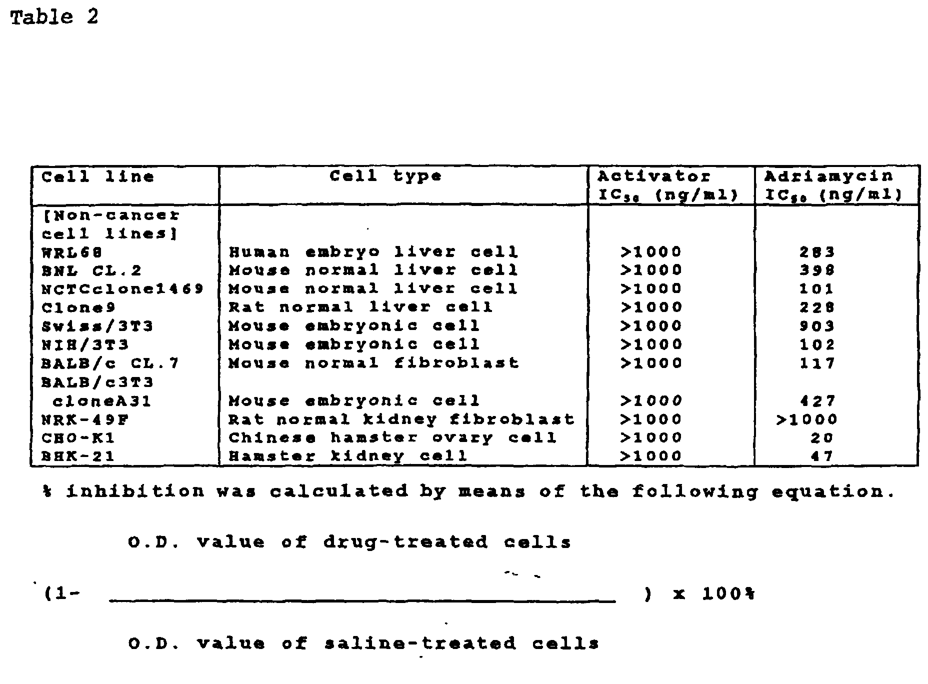

It is apparent from Tables 1 and 2 that the activator exhibited strong growth inhibitory effects on many epithelial and fibroblast-effects were fully comparable to the effects of adriamycin which shows anticancer activity through inhibition of nucleic acid synthesis. The activator had effects on cancer cells from any organ, thus showing no organ specificity. On the other hand, the activator of the invention did not inhibit growth of 4 liver-derived cell lines, even at the concentration of 1000 ng/ml. It should be noted that this in vitro anticancer effect was not observed at all with poly(I)·poly(C) alone or the carrier alone and that it is a phenomenon unaccountable from the mere translocation of poly(I)·poly(C) into the cells.

### Test Example 2: Observation of apoptosis

### (1) DNA and RNA fragmentation

### ① DNA fragmentation

To each of A431 cell line and KM12-HX cell line, 1 µg/ml of the activator of Example 4 was added. The A431 cells and KM12-HX cells were recovered after 5 hours and 7.5 hours, respectively. The cells were lyzed with 5 mM Tris-HCl (pH 8.0)-10 mM EDTA-0.5% (v/v) Triton X-100 and centrifuged at 13000 x g for 20 minutes to isolate the fragmented DNA (supernatant) and the chromatin fraction (pellet). Then, 100 µg/ml of Rnase A was permitted to act on the supernatant at 37°C for 1 hour and, thereafter, 200 µg/ml proteinase K and 1% (w/v) SDS (sodium dodecyl sulfate) were added for reaction at 50°C for 1.5 hours. The fragmented DNA was extracted with phenol-chloroform and subjected to 1.8% agarose gel electrophoresis. As a result, DNA fragmentation was observed in both cell lines.

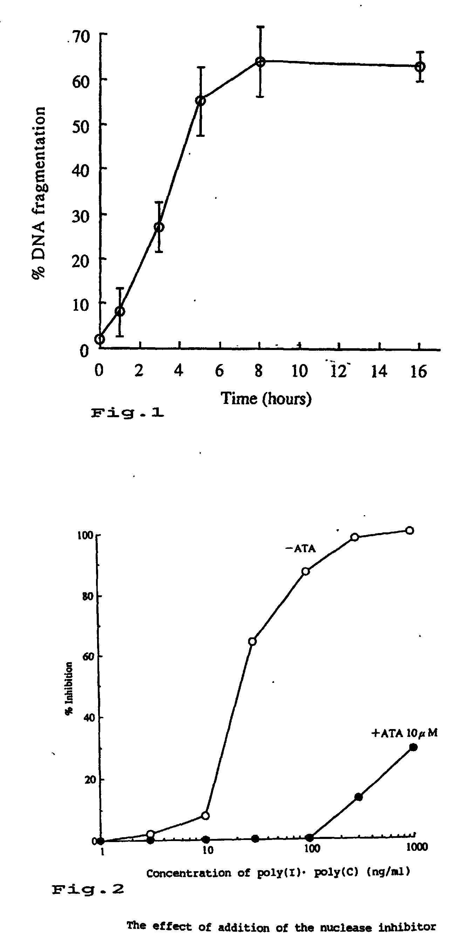

The time course of DNA fragmentation was investigated for A431 cell line. Thus, a 6-well plate was seeded with A431 cells at a density of 2.8x10⁵ cells/well and on the following day the cell DNA was labeled with 2 µCi[³H]thymidine. Then, the activator of Example 4 (1 µg/ml) was added and the cells were harvested at timed intervals. The cells were lyzed with 5 mM Tris-HCl (pH 8.0)-10 mM EDTA-0.5% (v/v) Triton X-100 and centrifuged at 13000 x g for 20 minutes to separate the fragmented DNA (supernatant) from the chromatin fraction (pellet). From the measured radioactivity in the supernatant and in the pellet, respectively, the ratio of fragmented DNA to total DNA was calculated. The results are shown in Fig. 1.

The fragmentation rate was about 30% of total DNA at 3 hours after addition and not less than 55% at 5 hours, indicating that this fragmentation occurs immediately following intracellular uptake of the activator of the invention.

### ② RNA fragmentation

To each of A431 cell line, MDA-MB-468 cell line, KB cell line, HeLa S3 cell line, and MCF-7 cell line, the activator of Example 4 as 1 µg/ml poly(I)·poly(C) was added, and the treated cells were recovered after 4 hours. From the recovered cells, the ribosome fraction was separated and the total RNA was extracted by the ACPC (Acid-Guanidium-Phenol-Chloroform) method. The RNA was subjected to formaldehyde-modified gel (1.8% agarose gel) electrophoresis and ethidium bromide staining. As a result, the fragmentation of 28S and 18S ribosome RNAs was observed in all the cell lines.

### (2) Effect of a nuclease inhibitor

A 96-well plate was seeded with HeLaS3 cells at a density of 10⁴ cells/well and on the following day 10 µM of the nuclease inhibitor ATA (aurintricarboxylic acid) and the activator according to Example 4 of the invention were simultaneously added. The cells were further grown for 3 days and the number of viable cells was determined by the MTT method. The results are shown in Fig. 2.

It is apparent from Fig. 2 that when the intracellular nuclease activity was inhibited by adding ATA, the activator of the invention failed to inhibit growth of cancer cells. Furthermore, when the ATA added was removed from the medium after 8 hours and before addition of the activator of the invention, the activator failed to express its activity. Therefore, it was thought that the effect of ATA was not that of inhibiting intracellular uptake of the activator but that of acting as a nuclease inhibitor.

### (3) The above test results indicate that the activator of the invention activates intracellular nucleases to thereby induce apoptosis of cancer cells.

### Test Example 3: Effect in the murine metastatic hepatocarcinoma model (in vivo)

Using nude mice Balb/c, nu/nu (aged 5 weeks, male), 10⁶ cells/mouse of KM12-HX cell line (a human colon cancer cell line which, when transplanted in the spleen of nude mice, metastatizes to the liver with high efficiency to cause neoplastic lesions) was injected into the spleen and after 10 minutes the spleen was enucleated. Starting 3 days later, the activator according to Example 4 of the invention was administered intravenously twice weekly at substantially constant intervals for 5 consecutive weeks. Two days after the last dose the liver was isolated and the number and area of cancer nodules formed in the liver were determined. The results are shown in Table 3.

**Table 3**

| | Number of cancer nodules | Area (mm²) |
|---|---|---|
| Control (10% maltose) | 35±6.2 | 244±44 |

| Activator of the invention | | |
|---|---|---|
| 30 µg/kg, twice weekly | 11±4.0* | 69±32 (72%)* |
| 100 µg/kg, twice weekly | 6.1±1.4* | 22±8.1(91%)* |
| Control (10% maltose) | 20±3.5 | 165±29 |

| Activator of the invention | | |
|---|---|---|
| 100 µg/kg, twice weekly | 3.9±0.8* | 4.2±1.0(97%)* |
| 100 µg/kg, once weekly | 6.4±1.7* | 38 ±16 (77%)* |
| The figures in the table denote the number and area of cancer nodules per mouse (mean ± S.E.). The figure in parentheses denote % inhibition. | | |

| | | |
|---|---|---|
| * : significant at p<0.01 (Dunnett's test). | | |

Compared with the control group (given 10% maltose), the hepatic cancer cell growth inhibition was 72% in the 30 µg/kg group and 91% in the 100 µg/kg group. In the 100 µg/kg group, 77% inhibition was obtained even when the dosage schedule was once a week.

In addition, liver tissue specimens were prepared and pathologically examined. As a result, the liver cancer in the control group was epithelial low-differentiation adenocarcinoma. The ordinary degree of feeder vascularization was observed. No remarkable immune cell infiltration was in evidence. Moreover, the tumorous tissue showed local calcification. In the group treated with the activator of the invention, no overt cancer cells were detected but only the calcification remaining after healing was noted.

Thus, the activator of the invention showed significant efficacy in the animal hepatocarcinoma model within the dose range of 10 µg/kg-100 µg/kg in a dosing schedule of twice a week.

### Test Example 4: Toxicity study

### (1) Expression of hepatotoxicity in rats given a single dose (acute toxicity study)

Using 8 male SD rats aged 6 weeks, the activator according to Example 4 of the invention was administered in a single intravenous dose and the serum aminoacyl transferase activity was determined after 20 hours. As a result, no death was encountered up to 5 mg/kg and a slight elevation of serum aminoacyl transferase at most was observed at 5 mg/kg. At 1 mg/kg, the serum aminoacyl transferase level was little elevated.

### (2) Two-week subacute toxicity study in rats

The activator according to Example 4 of the invention was administered intravenously to 6 male SD rats (aged 6 weeks) daily for 14 consecutive days. As a result, no remarkable sign of toxicity was found at doses up to 1 mg/kg.

### (3) Antigenicity study

Using male guinea pigs (Hartley strain, 5 weeks old), the antigenicity of the activator according to Example 4 of the invention was studied. As a result, no antigenicity was found at 50 µg/animal.

### (4) Expedient mutagenicity study

The activator according to Example 4 of the invention was subjected to expedient reverse mutation assay and expedient chromosomal aberration assay. As a result, no mutagenicity was found at 10 µg/ml.

### BRIEF DESCRIPTION OF THE DRAWINGS

Fig. 1 is a graph showing DNA fragmentation rates. The ordinate represents the percentage (%) of DNA fragmentation and the abscissa represents time (hr).
Fig. 2 is a graph showing the effect of addition of the nuclease inhibitor ATA, wherein the abscissa represents the concentration (ng/ml) of the activator according to Example 4 of the invention, -○- r epresents the data generated in an ATA-free system, and -●-represents the data generated in a system with ATA.

## Claims

1. A cancer cell nuclease activator comprising a complex of a carrier effective in intracellular delivery of a drug with poly(I)· poly(C), mismatched poly(I)·poly(C), poly(A)·poly(U) or mismatched poly(A)·poly(U).

2. The cancer cell nuclease activator according to Claim 1 wherein the carrier effective in intracellular delivery of a drug is a cationic liposome preparation.

3. A cancer cell nuclease activator comprising a complex of a carrier essentially consisting of 2-O-(2-diethylaminoethyl)carbamoyl-1,3-O-dioleolylglycerol and a phospholipid with poly(I)·poly(C) or mismatched poly(I)·poly(C).

4. The cancer cell nuclease activator according to Claim 3 wherein the phospholipid is lecithin.

5. The cancer cell nuclease activator according to Claim 3 or 4 wherein the poly(I)·poly(C) has a mean chain length within the range of1 00 to 500 bp.

6. A cancer cell nuclease activator.

7. The cancer cell nuclease activator according to any of Claims 3 through 5 which the cancer is hepatocarcinoma.

8. An anticancer composition comprising the cancer cell nuclease ctivator claimed in any of Claims 1 through 7.
